# EUROPEAN PATENT APPLICATION

(11) **EP 1 138 323 A2**
(43) Date of publication of application: **04.10.2001**
(21) Application number: 01106638.8
(22) Date of filing: 16.03.2001
(51) Int. Cl.: A61K 31/047, A61K 9/06, A61P 17/00, A61P 17/06, A61P 17/10

(54) **Resveratrol for the treatment of exfoliative eczema, acne or psoriasis**

(30) Priority: 24.03.2000 IT MI000630
(71) Applicant: D.B.P. (Development Biotechnological Processes) di Rossi Valentina e C. s.n.c., 83100 Avellino (IT); Nuova ICT s.r.l., 26845 Codogno (Lodi) (IT)
(72) Inventor: Pelliccia, Maria Teresa, 83100 Avellino (IT); Giannella, Attilio, 26845 Codogno (Lodi) (IT); Giannella, Jenny, 26845 Codogno (Lodi) (IT)
(74) Representative: Minoja, Fabrizio, Dr.

(57) **Abstract**

The use of resveratrol (3,4',5-trihydroxy-trans-stilbene) and derivatives thereof, for the preparation of medicaments for the treatment of exfoliative eczema, acne and psoriasis, topical pharmaceutical formulations containing resveratrol or derivatives thereof in combination with other active principles. Treatment consists in topical administrations of resveratrol at concentrations of 0.01 to 20%, in the form of lotions, creams or ointments, optionally in combination with other active principles such as melatonin, vitamins D, E and A and derivatives thereof, hormones, vegetable and/or animal extracts. Contrary to current therapies, the use of resveratrol has neither systemic nor topical effects during and after therapy.

## Description

The present invention relates to the use of resveratrol (3,4',5-trihydroxy-trans-stilbene) and derivatives thereof (esters, glycosides, 3'-oxyresveratrol), for the preparation of medicaments for the treatment of exfoliative eczema, acne and psoriasis.

Resveratrol (3,4',5-trihydroxy-trans-stilbene), a phytoalexin produced by a number of vegetables under stress conditions, is one of the natural substances of vegetable origin at present arousing great interest in the pharmaceutical, cosmetic and nutritional fields, due to the important, established effects this molecule exerts in humans.

In vitro and in vivo studies on resveratrol proved that the molecule: a) exerts protective action on the cardiovascular system, (Clin. Chim. Acta, 235:207, 1995) and decreases arteriosclerosis risks (Clin. Chim. Acta, 246:163, 1996); b) has vasal relaxing effect on the arteries (Gen. Pharm. 27:363, 1996); c) has antioxidant action which inhibits LDL cholesterol peroxidation (The Lancet, 341: 1103, 1993); reduces oxidative stress (Neuroreport 8:1499, 1997); protects from the radical damage in cerebral ischemia (Chin. Pharm. Bull. 12:128, 1996); prevents the propagation of free radicals responsible for the molecular damage of the biological systems and for cell aging; d) modulates lipid synthesis, preventing the accumulation of cholesterol and fats in the liver, decreases the concentrations of blood triglycerids and of cholesterol in low-density LDL lipoproteins and reduces the atherogenic index (Chem Pharm. Bull. 30:1766, 1982); e) inhibits platelet aggregation, preventing the formation of thrombi (Int. J. Tiss. Reac. XVIII, 1, 1995; Thrombosis and Haemostasis, 76: 818, 1996); f) inhibits the production of pro-atherogenic eicosanoids by platelets and neutrophils, exerting anti-inflammatory action (Biochem. Biophys. Acta, 834:275, 1985); g) inhibits protein-tyrosine kinase which modulates cell proliferation and differentiation and the signaling processes in the immune system cells, biological processes involved in the inflammatory response and in severe pathologies such as cancer, arteriosclerosis and psoriasis (J. Natural Products, 56:1805, 1993, Science 267:1782, 1995); h) has marked antimutagenic action, inhibiting the cell events connected with the initiation, promotion and progression of the tumor (Science 275:218, 1997, Anal. Biochem, 169:328, 1988, Proc. Natl. Acad. Sci USA, 91:3147, 1994, Proc. Natl. Acad. USA, 72:1848, 1975, Carcinogenesis, 8:541, 1987). The presence of resveratrol traces in red wines is believed to be the main cause of the beneficial nutritional effects thereof (Am, J. Enol. Vitic. 46:159, 1996, Clin. Chim. Acta, 246:183, 1996, Amer. J. Clin. Nutr., 55:1012, 1992). The poor concentrations of resveratrol in wine and in wine industry by-products have, until some time ago, remarkably restricted a wide use of this molecule in the pharmaceutical and nutritional fields. Recently, rhizomes of the Chinese plant Poligonum cuspidatum have been found to contain high amounts of resveratrol (more than about 400 times those in wine) thus inducing a strong commercial development of this molecule as alimentary supplement, in particular on the U.S. market. Lately, the actions of resveratrol for pharmacological or cosmetic use have been claimed (WO9959561; WO9958119; EP0773020; FR2766176; WO9904747).

It has now been found that resveratrol can be effectively used in the treatment of exfoliative eczema, acne, psoriasis lesions and all the exfoliative skin diseases. The treatment according to the invention consists in the topical administration of resveratrol at concentrations of 0.01 to 20%, preferably of 1 to 5%, in the form of lotions, creams or ointments, also in combination with other active principles such as melatonin, vitamins D, E and A or derivatives thereof, hormones, vegetable and/or animal extracts, azadirachtin, retinoic acid or derivatives thereof, methotrexate or derivatives thereof, cyclosporin or derivatives thereof, palladium and/o ruthenium or derivatives thereof, immunosuppressors, anti-inflammatory agents, phototherapeutics and cell hyperproliferation modulators.

Particularly preferred are the combinations with vitamin D derivatives and with corticoids for the topical treatment of psoriasis, and with vitamin A for the treatment of skin dystrophic forms possibly associated with scaling.

Contrary to the current therapies, the use of resveratrol induces neither systemic nor topical effects during and after the treatment. Furthermore, no changes of neutrophils activity, of circulating cells or of cells at the lesion site, were observed after topical treatment with resveratrol. Therefore, the therapeutical action of resveratrol cannot be ascribed at all to an already known action mechanism connected with the neutrophil component (Biochem. Biophys. Acta, 834:275, 1985).

As a consequence, none of the well-known above described properties of resveratrol can explain the effectiveness of the molecule in the treatments of exfoliative eczema, acne, psoriasis and generally all exfoliative skin diseases, which is the object of the present application.

Therefore, none available knowledge of resveratrol could suggest its usefulness in the treatment of the diseases according to the invention. In particular, WO9959561 (Hensley et al.) considers the action on neutrophils, particularly on the enzyme myeloperoxidase, the therapeutical basis of the use of resveratrol in a series of diseases, inter alia psoriasis itself. As a matter of fact, psoriasis pathogenesis is much more complex and cannot at all be ascribed to the only action of neutrophils, or even more simplistically to the activation of a single enzyme, myeloperoxidase, produced by said cells. On the other hand, the involvement of various types of cells, either immunocompetent or not, in psoriasis is widely established (Nature Medicine 5, 442, 1995; J. Invest. Dermatol. 101, 695, 1993; J. Invest. Dermatol. 102, 145, 1994) and the treatments used to date act on both the immunologic and keratinocyte components.

Current treatments for some of the pathologies for which the topical use of resveratrol is proposed, as well as the limits thereof, are summarized in the following.

### EXFOLIATIVE ECZEMA

Exfoliative eczema is a skin inflammation characterized by redness, itching and oozing, sometime scaling, lesions. As for the etiology, family history of atopy is frequent (Journal of Allergy and Clinical Immunology, 13, 487-494, 1986). In any event, the development of said skin allergic sensitivity definitely involves environmental triggering factors. In 1988 some researchers have identified a significant connection between atopy and chromosome 11q13 (Lancet 1, 1292-1295, 1988). The treatment of exfoliative eczema may be extremely demanding and the severity of symptoms, the age of the patient, the affected body site and any other worsening factors should be considered. No treatment exists which can ease symptoms in all of the treated patients. To date, most therapies, either the topical and/or systemic ones, may involve remarkable side effects thus making it necessary to stop treatment. Topical treatment of exfoliative eczema comprises the use of emollients, steroids (which can induce atrophy and depigmentation of the skin as well as iatrogenic Cushing's syndrome in case of systemic absorption) and topical or systemic immunosuppressors (cyclosporin, FK-506 or other similar drugs inducing severe side effects). Furthermore, a number of studies proved the beneficial effect of ultraviolet radiations in the treatment of exfoliative eczema. Exposure to UV rays, however, involves risks of development of skin cancer and should therefore be considered a last resort.

### ACNE

Acne is a disease of the pilosebaceous follicle connected with disfunctions of the sebaceous gland. Development from sebum overproduction to acne takes place when in sebaceous follicles, which have a large sebaceous gland and a thin hair, hyperkeratosis of the hair follicle infrainfundibular portion occurs, most likely following an irritant stimulus on the infrainfundibular keratinic cells. This leads to blockage of the sebaceous follicle, thus preventing sebum discharge. These conditions are extremely favorable to the growth of some bacteria, such as Propionibacterium acnes. The increase in the sebum mass, causes dilation and rupture of the follicle with the release of its contents into the dermal tissue, with severe inflammation. As a consequence appear the typical acne lesions: papules, pustules and, in the more serious cases, nodules and cysts. The therapeutical approach should take into consideration the severity of the process, the polymorphism of the lesions as well as the complex etiology of the disorder. Substances currently available for the topical or systemic treatment of acne (benzoyl peroxide, retinoic acid, azelaic acid, oral antibiotics, anti-androgens) can involve remarkable side effects, so that occasionally treatment must be discontinued.

### PSORIASIS

Psoriasis is a common inflammatory disease of the skin characterized by excessive scaling, which affects about 2% of the population (Cristophers, E, and Sterry W, 1993. Psoriasis. In Dermatology in General Medicine. T.B. Fitzpatrick et al editors. McGraw-Hill , New-York, 489-514 ). Etiology is still unknown, and the primary cause could be due either to abnormal keratinocytes function or to inflammatory-immune disorders. Psoriasis varies in type and severity and can affect different body areas. Current therapies can be topical and/or systemic, but all of them involve remarkable side effects which can induce discontinuation of the treatment. Topical treatments with mineral coaltar (which has unpleasant smell, uncertain effectiveness and induces photosensitization), with steroids (whose topical and systemic side effects are well known), vitamins D3 and retinoids (which can cause photosensitization and are teratogenic) have been proposed. Furthermore, treatment with ultraviolet radiation optionally combined with the administration of psoralenes has been suggested. Drugs used for the systemic therapy of psoriasis comprise: methotrexate, cyclosporin and other immunosuppressors, oligoelements such as palladium or red ruthenium and antiproliferative drugs, all of them having unnegligeable toxic potential.

The invention is described in further detail in the following.

### Example 1 - Exfoliative eczema

Experiments were carried out on patients (n=20) of both sexes, of age above 18 years, suffering from severe disabling exfoliative eczema unresponsive to the current topical treatments.

All patients were subjected to complete blood count and measurements of renal and hepatic functions prior to treatment. Patients who had been systemically treated two months previously with corticoids, antibiotics, psoralenes and other immunosuppressants or with UV were excluded. Patients were supposed not to use any corticoid topically.

Treatment - Out of 20 patients, 10 were included in the resveratrol-treated group (1% resveratrol ointment) and 10 in the control group (ointment with no resveratrol). The two groups were comparable as for sex, age, duration and severity of the eczema. Patients were divided in two groups and treated twice a day for six months either with the resveratrol-containing ointment or with the placebo ointment (control group).

Evaluations - The progress of the disease was monitored by using the "six-area/six-sign score" method. The severity of the six signs used for monitoring the disease (erythema, pustules, excoriations, dehydration, scaling of the skin and lichenification), was evaluated on a 0-3 score (none, mean, moderate and severe), in each of the six body areas tested (head, neck, hands, elbows, feet, legs and trunk). The severity of itching and of sleep disorders was evaluated on a 0-3 score (none, mean, moderate and severe).

Results - Only in the resveratrol-treated subjects the mean of the values concerning the different clinical symptoms considered rapidly decreased from 57.0 (SEM 1.6 n=10) to 21.5 during the first two weeks of treatment. At the end of the treatment the 8 resveratrol-treated patients showed significant improvements of all the considered parameters, whereas two of them only showed improvements concerning skin scaling. No control subjects showed recovery signs. At the beginning of the treatment, the body area affected by eczema was 69% on the average in all patients. This gradually decreased during treatment, to reach 27% only in the resveratrol-treated patients. At the end of the treatment, the mean scores for itching decreased from 2.3 to 0.6 and for sleep disorders from 2.9 to 1.0. only in the resveratrol-treated patients.

### Example 2 - Acne

A randomized, double blind study was carried out in order to evaluate the effectiveness of topical administration of resveratrol in the treatment of acne vulgaris. Patients (n=30) of 15 to 19 years were all suffering from II or III degree acne (according to Pillsbury's classification), with multiple inflammatory lesions (20 to 62) and a number of comedos (28 to 120) on face and forehead. Only subjects who had received no systemic treatment with antibiotics for at least 4 weeks and had used no topical medicaments for 2 weeks before treatment were selected. During treatment with resveratrol, no further topical treatment was allowed. The effectiveness of the therapy was evaluated by comparing the conditions of the lesions before and after the treatment, according to an arbitrary score of 0 to 5 (0=worsening; 1=no changes; 2=slight improvement; 3=modest improvement; 4=good improvement; 5=excellent improvement).

Treatment - Treatment consisted of daily topical administrations of resveratrol (1% cream) or of the carrier only, for 10 weeks in 30 patients suffering from acne vulgaris. The evaluated parameters were: number of acne lesions and comedos, erythema, seborrhoic skin and skin scaling.

Results - Patients treated with resveratrol showed neither systemic nor topical side effects. A significant reduction of erythema, number of acne lesions, comedos and, above all, scaling, was observed in treated subjects (96%), compared with control subjects (2%). More particularly, the effectiveness of the treatment in 95% of cases proved to be due to the reduction of follicle hyperkeratosis, which is an important factor in the formation of comedos since it causes thickening of the follicle wall with sebum retention.

### Example 3 - Psoriasis

A prospectic, multi-center, double bind study was effected on patients of both sexes, of age above 18 years, with a clinical diagnosis of mild to moderate chronic psoriasis, with affected areas of at least 100 cm², but not above 40% of the total body area. Patients with pustular psoriasis or with psoriasis guttata, those who had received either topical treatment for two weeks before the test or systemic treatment in the 8 weeks before the test, as well as pregnant and nursing women or patients receiving more than 400 IU of vitamin D daily or any other medicament potentially affecting the progress of the disease, were excluded.

Treatment - Out of 48 patients, 12 were included in the resveratrol-treated group (1% resveratrol ointment), 12 in the control group (ointment with no resveratrol), 12 in a group treated with a Vitamin D derivative (calcipotriol 50 mg/g) and 12 in a group treated with the combination resveratrol/Vitamin D derivative (1% resveratrol / 50 mg/g calcipotriol). The 4 groups were comparable as for sex, age, duration and severity of psoriasis. Patients were treated twice a day for one month with the different ointments.

Evaluations- At the end of the treatment, the clinical response was evaluated as "healed", "marked improvement", "slight improvement", "no changes", "worsening". Quality life evaluations were carried out before and after the treatment, by using the "psoriasis Disability Index" (PDI) and the "Sickness Impact Profile" (SIP). The PDI questionnaire included 15 questions as follows: daily activities (5 questions), work or school, if applicable, (3 questions), personal relationships, (2 questions), spare time (4 questions), treatment, (1 question). Each question had a 1 to 7 score. The purpose of said questions was to evaluate symptoms and feelings of the patients concerning their psoriasis during the first 4 weeks of treatment.

Clinical effectiveness - At the end of the treatment, the percentages of patients healed or showing marked improvement were 80% for those treated with resveratrol and 10% for the control group. Effectiveness of the treatment with Vitamin D derivatives was observed only in 47% of the patients, whereas the Vitamin D derivatives / resveratrol combination was effective in 95% of patients.

Life quality of the patients, as evaluated by PDI, increased only after treatment with resveratrol. The reduction in the total PDI score was 6.5 in the resveratrol group (95% CI 4.4, 8.6; P=0.001 ) and 1.7 in the control group (95% CI 1.1, 6.3 ; P= 0.005).

## Claims

1. The use of resveratrol or derivatives thereof for the preparation of medicaments for the treatment of exfoliative eczema and hyperkeratosis disorders.

2. The use of resveratrol or derivatives thereof for the preparation of medicaments for the treatment of acne.

3. The use of resveratrol or derivatives thereof for the preparation of medicaments for the treatment of psoriasis.

4. Topical pharmaceutical formulations containing resveratrol or derivatives thereof in combination with melatonin, vitamins D, E and A or derivatives thereof, hormones, vegetable and/or animal extracts, azadirachtin, retinoic acid or derivatives thereof, methotrexate or derivatives thereof, cyclosporin or derivatives thereof, palladium and/o ruthenium or derivatives thereof, immunosuppressors, anti-inflammatory agents, phototherapeutics and cell hyperproliferation modulators.

5. Formulations as claimed in claim 4 containing 0.01 to 20% of resveratrol and/or derivatives thereof.

6. Formulations as claimed in claim 5 containing 1 to 5% of resveratrol and/or derivatives thereof.

7. Formulations as claimed in claims 4 to 6, wherein resveratrol derivatives are selected from the esters and glycosides thereof, and 3'-oxyresveratrol.

8. Formulations as claimed in claims 4 to 7 in the form of gel, creams or ointments.
